(19) Europäisches Patentamt
European Patent Office
Office européen des brevets

(11) Publication number: **0 522 157 A1**

(12) **EUROPEAN PATENT APPLICATION published in accordance with Art. 158(3) EPC**

(21) Application number: **91903583.2**

(51) Int. Cl.5: **A61K 7/06**

(22) Date of filing: **12.02.91**

(86) International application number: **PCT/JP91/00159**

(87) International publication number: **WO 91/11983 (22.08.91 91/19)**

(30) Priority: **13.02.90 JP 31737/90**

(43) Date of publication of application: **13.01.93 Bulletin 93/02**

(84) Designated Contracting States: **AT BE CH DE DK ES FR GB GR IT LI LU NL SE**

(71) Applicant: **TAISHO PHARMACEUTICAL CO. LTD**
**24-1 Takata 3-chome Toshima-ku**
**Tokyo 171(JP)**

(72) Inventor: **MITSUYAMA, Syunpei**
**Nishi-ageodainidanchi 1-8-306, 77-1**
**Oaza Koshikiya, Ageo-shi, Saitama 362(JP)**
Inventor: **NAGASHIMA, Shin-ichi**
**202, Sanraitodaido, 1985-5, Kawarabuki**
**Ageo-shi, Saitama 362(JP)**
Inventor: **KIMURA, Michiko**
**207-1, Ofukuda, Goka-mura**
**Sashima-gun, Ibaraki 306-03(JP)**
Inventor: **URUSHIZAKI, Fumio**
**A1110, Sofia-ageo, 2-1-1, Yatsu**
**Ageo-shi, Saitama 362(JP)**
Inventor: **YOSHIDA, Tsuguchika**
**713-11, Ageo-mura**
**Ageo-shi, Saitama 362(JP)**

(74) Representative: **Lamb, John Baxter et al**
**MARKS & CLERK 57-60 Lincoln's Inn Fields**
**London WC2A 3LS(GB)**

# (54) NOVEL USE OF ALKANAMIDOCARBOXYBETAINE.

(57) An alkanamidocarboxybetaine of formula (I) or its salt, which is effective in stimulating hair growth of a mammal, wherein $R^1$ represents an alkyl with 5 to 25 carbon atoms; $R^2$ and $R^3$ represent each an alkyl with 1 to 4 carbon atoms; A represents an alkylene with 1 to 3 carbon atoms optionally substituted by methyl or hydroxyl; and m is an integer of 1 to 3.

$$R^1-\overset{\displaystyle O}{\overset{\|}{C}}-\overset{\displaystyle H}{\overset{|}{N}}-(CH_2)_m-\underset{\displaystyle R^3}{\underset{|}{\overset{\displaystyle R^2}{\overset{|}{N^+}}}}-A-COO^- \quad (1)$$

EP 0 522 157 A1

## TECHNICAL FIELD

The present invention relates to a new use of alkanamidocarboxybetaines, and more particularly relates to a new use of alkanamidocarboxybetaines for promoting hair generation of mammals including humans.

## BACKGROUND ART

In order to improve alopecia in mammals, there have heretofore been used substances having the hair generation promoting effect such as, for example, calpronium chloride, estradiol, swertiamarin, glycerol pentadecanate and gineseng extract. However, many of these substances are not sufficiently effective, and even though other substances may have the remarkable effect, they cannot withstand practical use for the skin irritation property. Accordingly, alopecia cannot be significantly improved by the prior art.

As a result of the various researches to improve alopecia, the present inventors have found that alkanamidocarboxybetains have an excellent hair generation promoting effect as well as a low skin irritant property, and have accomplished the present invention based on this finding.

On the other hand, certain alkanamidocarboxybetaines are known to be incorporated into shampoos as surfactants in Japanese Patent Kokai No. 60-197614 and ibid. 60-132912, but not found to have a hair generation promoting effect.

## DISCLOSURE OF INVENTION

An object of the present invention is to provide a method for promoting hair generation of a mammal comprising topically applying an effective amount of an alkanamidocarboxybetain represented by the formula;

$$R^1-\overset{O}{\overset{\|}{C}}-\overset{H}{\overset{|}{N}}-(CH_2)_m-\underset{\underset{R^3}{|}}{\overset{\overset{R^2}{|}}{N^+}}-A-COO^- \qquad (I)$$

(wherein $R^1$ is an alkyl group having 5 to 25 carbon atoms, $R^2$ and $R^3$ are each an alkyl group having 1 to 4 carbon atoms, A is an alkylene group having 1 to 3 carbon atoms, or an alkylene group having 1 to 3 carbon atoms substituted by a methyl group or a hydroxyl group, and m is an integer of 1 to 3) or a salt thereof.

A further object of the present invention is to provide a hair generation promoting agent comprising an alkanamidocarboxybetaine of Formula (I) or a salt thereof as an effective ingredient.

A still further object of the present invention is to provide a use of an alkanamidocarboxybetaine of Formula (I) or a salt thereof for the manufacture of a hair generation promoting agent.

In the present invention, the alkyl group having 5 to 25 carbon atoms refers to, for example, a pentyl group, a hexyl group, a heptyl group, an octyl group, a nonyl group, a decyl group, an undecyl group, a dodecyl group, a tetradecyl group, a pentadecyl group, a hexadecyl group, an octadecyl group, an eicosyl group and a tetracosyl group, and may be arranged as straight or branched chains.

The alkyl group having 1 to 4 carbon atoms refers to, for example, a methyl group, an ethyl group, a propyl group and a butyl group, and may be arranged as straight or branched chains.

Among the preferred compounds of Formula (I) are N-[2-(octanamido)ethyl]-N,N-dimethylammonioacetate, N-(cocoalkanamidomethyl)-N,N-dimethylammoniobutyrate, N-[2-(hexadecanamido)ethyl]-N,N-dimethylammoniobutyrate, N-[3-(cocoalkanamido)propyl]-N,N-dimethylammonioacetate, N-[3-(docosanamido)propyl]-N,N-dimethylammonioacetate, N-[2-(tetracosanamido)ethyl]-N,N-dimethylammonioacetate, N-(icosanamidomethyl)-N,N-dimethylammonioacetate, N-(hexadecanamidomethyl)-N,N-dimethylammonioacetate and N-[3-(hexadecanamido)propyl]-N,N-dimethylammonioacetate. Most preferred among them are N-[3-(docosanamido)-propyl]-N,N-dimethylammonioacetate and N-[3-(cocoalkanamido)-propyl]-N,N-dimethylammonioacetate.

As the effective ingredient of the present invention, one or more compounds of Formula (I) or the salt thereof can be used. The effective ingredient can be topically applied in the dosage forms such as lotions, emulsions, creams, gels and aerosols, all of which can be prepared by usual methods, for example, the

method described in the Pharmacopoea of Japan, 11th Edition. The amount of the effective ingredient is 0.05 - 30% by weight, preferably 0.5 - 5% by weight based on the total formulation.

The preparation thus obtained can be applied to the scalp several times a day in a suitable amount.

Into the hair generation promoting agent of the present invention can be incorporated, besides the above-mentioned effective ingredient, substances used for ordinary hair generation promoting agents, for example, solvents (e.g. ethyl alcohol, isopropyl alcohol, 1,3-butylene glycol, 1,4-butylene glycol, propylene glycol, dipropylene glycol, glycerol, diglycerol, polyethylene glycol and purified water), mucopolysaccarides (e.g. hyaluronic acid, condroitin-4-sulfate, condroitin-6-sulfate, dermatan sulfate, keratan sulfate, heparan sulfate and condroitin polysulfate), preservatives (e.g. parabens and benzoic acid), vitamins [e.g. vitamin A, vitamin $B_1$, vitamin $B_2$, vitamin $B_6$, vitamin C, vitamin D, vitamin E and derivatives thereof (e.g. vitamin E acetate)], oils (e.g. liquid paraffin, white soft paraffin, solid paraffin, ceresin, microcrystalline wax, cholesterol, squalene, olive oil, rose hip oil, mink oil, jojoba oil, hydrogenated castor oil, hydrogenated coconut oil, isopropyl myristate, ethyl caproate, ethyl caprylate, palmitoleic acid, ethyl palmitolate, ethyl linolate, ethyl linolenoate, oleic acid, stearic acid, linoleic acid, linolenic acid, palmitic acid, behenic acid, lauric acid, stearyl alcohol, cetyl alcohol, lauryl alcohol, oleyl alcohol and cetyl isooctanoate), germicides [e.g. sulfur, chlorhexidine gluconate, chlorhexidine hydrochloride, cetyl pyridinium chloride, dequalinium chloride, isopropyl methyl phenol, quaternary ammonium salts (e.g. benzalkonium chloride) and hinokitiol], nonionic surfactants (e.g. polyoxyethylene fatty acid esters, polyoxyethylene sorbitan fatty acid esters, sorbitan fatty acid esters, glycerol fatty acid esters, polyoxyethylene glycerol fatty acid esters, polyglycerol fatty acid ester, propylene glycol fatty acid esters, polyglycerol alkyl phenyl ethers, polyoxyethylene hydrogenated castor oil and copolymers of polyoxyethylene and polypropylene glycol), anionic surfactants (e.g. N-acylamino acid salts, N-acylsarcosine salt, alkylphosphate salt and acylmethyltaurine salt), cationic surfactants (e.g. alkyltrimethyl ammonium and alkyl-N,N-dialkylaminoacetic acid esters), amphoteric surfactants (e.g. imidazoline and amineoxide), high-molecular surfactants (e.g. casein), silicone derivatives (e.g. silicone oil, polyol denaturated silicone and silicone resin), thickners (e.g. methylcellulose, polyvinylpyrrolidone, carboxymethylcellulose, carboxyethylcellulose, hydroxypropylcellulose and carboxyvinyl polymers), clay minerals (e.g. montmorillonite, saponite and hectolide), pH regulators (e.g. diisopropanolamine and citric acid), anti-oxidants (e.g. dibutylhydroxytoluene, potassium hydrogen sulfite, catechin and gluconodeltalactone), whitening agents (e.g. albutin and kojic acid), refrigerants (e.g. 1-menthol and camphor), anti-inframmatory agents (e.g. glycyrrhetinic acid, dipotassium glycyrrhizinate, berberine chloride, shikonin, guaiazulene, allantoin and $\delta$-aminocaproic acid), peripheral vasodilators (e.g. methyl nicotinate, benzyl nicotinate, swertiamarin, minoxidil, diazoxide, capsicum extract, capsicin and calpronium chloride), adrenocortical hormones (e.g. hydrocortisone acetate and betamethasone valerate), antihistamines (diphenhydramine hydrochloride and isothipendyl hydrochloride), local anesthetics (e.g. dibucaine hydrochloride and lidocaine hydrochloride), keratolytics (e.g. urea and salicylic acid), keratoregulators [e.g. vitamin A acid and derivative thereof (e.g. 13-cis-retinoic acid and etoretinate)], estrogens (e.g. 17$\beta$-estradiol, ethynylestradiol and estrone), progestins (e.g. progesterone and 17$\alpha$-hydroxyprogesterone acetate), anti-androgens (e.g. cyproterone acetate and 4-androstene-3-one-17$\beta$-carboxylic acid), perfumes, sequestering agent, ultraviolet absorbents, moistening agents (e.g. snake gourd extract, ginseng extract, diisopropyl acetate, pyrrolidone carboxylic acid, polyglutamic acid, polyoxyalkylenealkylglycoside ether, collagen, lecithin, ceramide and 2-aminoethanesulfonic acid) and crude drug extracts (e.g. cashew extract, panax rhizome extract, lansic extract and saffran extract). The amounts of the substances are not so much as to degrade the effect of the present invention.

BEST MODE OF CARRYING OUT THE INVENTION

The present invention is further illustrated in more detail by the following preparation of a compound of Formula (I), examples and experiment.

(Preparation)

N-[3-(Tetracosanamido)propyl]-N,N-dimethylammonioacetate

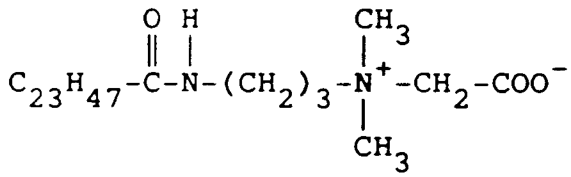

Ten ml of isopropyl alcohol and 5 ml of water were added to a mixture of 2.00 g of N-[3-(dimethylamino)propyl]tetracosanamide, 0.64 g of sodium chloroacetate and 0.04 g of sodium hydrogencarbonate, and the mixture was refluxed with heating for 6 hours. The resulting product was purified by silica gel column chromatography to give 1.37 g of the title compound.

$^1$H-NMR (methanol-$d_4$, $\delta$ ppm):

0.89(3H, m), 1.28(40H, s), 1.52-1.69(2H, m), 1.85-2.02(2H, m), 2.18(2H, t, J=8Hz), 3.18-3.28(8H, m), 3.53-3.67(2H, m), 3.78(2H, s)

Other novel compounds among those of Formula (I) can be also prepared according to the foregoing preparation.

(Example 1)

| Ingredients | Amounts |
| --- | --- |
| N-[2-(Octaneamido)ethyl]-N,N-dimethylammonioacetate | 3 g |
| Glycerol | 5 g |
| Dibutylhydroxytoluene | 0.1 g |
| Chlorhexidine gluconate | 0.1 g |
| Vitamin $B_6$ | 0.5 g |
| Polyoxyethylene monostearate | 1.0 g |
| Methylcellulose | 0.2 g |
| Dipotassium glycyrrhizinate | 0.2 g |
| Capsicum tincture | 0.1 g |
| Purified water | Total 100 g |

The ingredients were mixed and dissolved in the solvent to give a lotion.

(Example 2)

| Ingredients | Amounts |
| --- | --- |
| N-(Cocoalkanamidomethyl)-N,N-dimethylammonioacetate | 2.5 g |
| 1,3-Butyleneglycol | 5 g |
| Hydrocortisone acetate | 0.0016 g |
| Isothipendyl hydrochloride | 0.1 g |
| Sulfur | 3.0 g |
| Estrone | 0.0008 g |
| Ethyl alcohol | 20 g |
| Purified water | Total 100 g |

A lotion was prepared in a similar manner to that of Example 1.

(Example 3)

| Ingredients | Amounts |
|---|---|
| N-[2-(Hexadecanamido)ethyl]-N,N-dimethylammonioacetate | 5 g |
| Glycyrrhetinic acid | 2 g |
| Propylene glycol | 3 g |
| N-Decyl-N,N-dimethylammonioacetate Polyoxyethylene hydrogenated | 3 g |
| castor oil | 3 g |
| Isopropyl alcohol | 5 g |
| Purified water | Total 100 g |

A lotion was prepared in a similar manner to that of Example 1.

(Example 4)

| Ingredients | Amounts |
|---|---|
| N-[3-(Cocoalkanamido)propyl]-N,N-dimethylammonioacetate | 1 g |
| Dipotassium glycyrrhizinate | 1 g |
| Minoxidil | 0.5 g |
| Ethyl alcohol | 20 g |
| Propylene glycol | 2 g |
| Purified water | Total 100 g |

A lotion was prepared in a similar manner to that of Example 1.

(Example 5)

| Ingredients | Amounts |
|---|---|
| N-[3-(Docosanamido)propyl]-N,N-dimethylammonioacetate | 2 g |
| 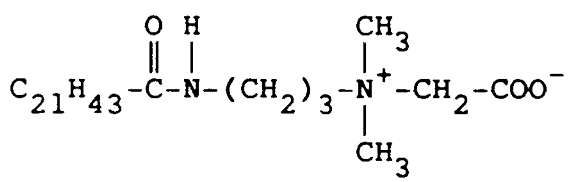 | |
| Ethanol | Total 100 g |

A lotion was prepared in a similar manner to that of Example 1.

(Example 6)

| Ingredients | Amounts |
|---|---|
| N-[2-(Tetracosanamido)ethyl]-N,N-dimethylammonioacetate | 3 g |
| Vitamin E acetate | 0.2 g |
| Glycyrrhetinic acid | 0.2 g |
| Glycerol monocaprate | 1 g |
| Squalene | 5 g |
| Liquid paraffin | 15 g |
| White soft paraffin | 3 g |
| Polyoxyethylene(20) sorbitan monostearate | 4 g |
| Purified water | Total 100 g |

The oil-soluble ingredients and the nonionic surfactant were dissolved with warming in an oily phase containing squalene, liquid paraffin and white soft paraffin. Thereto was added a water phase obtained by dissolving N-[2-(tetracosanamido)ethyl]-N,N-dimethylammonioacetate and the water-soluble ingredients with warming for emulsification, and the mixture was cooled to give a cream.

(Example 7)

| Ingredients | Amounts |
|---|---|
| N-(Icosanamidomethyl)-N,N-dimethylammonioacetate | 4 g |
| Squalene | 8 g |
| Stearyl alcohol | 2 g |
| Polyoxyethylene hydrogenated castor oil | 5 g |
| Polyoxyethylene sorbitan monostearate | 2 g |
| Benzalkonium chloride | 0.1 g |
| Purified water | Total 100 g |

The oil-soluble ingredients and the nonionic surfactants were dissolved with warming in an oily phase containing squalene and stearyl alcohol. Thereto was added a water phase obtained by dissolving N-(icosanamidomethyl)-N,N-dimethylammonioacetate and the water-soluble ingredients with warming for emulsification, and the mixture was cooled to give an emulsion.

(Example 8)

Base Liquid

| Ingredients | Amounts |
|---|---|
| N-(Hexadecanamidomethyl)-N,N-dimethylammonioacetate | 3 g |
| Squalene | 2 g |
| Stearyl alcohol | 0.2 g |
| Polyoxyethylene hydrogenated castor oil | 1 g |
| Polyoxyethylene sorbitan monostearate | 0.5 g |
| Parabens | 0.15 g |
| Purified water | Total 100 g |

Fillers

Dimethyl ether

The base liquid was prepared according to the process for the preparation of the emulsion in Example 7, and an aerosol agent was prepared by the following formulation in a usual filling manner.

| Formulation | |
|---|---|
| Base liquid<br>Filler | 30% by weight<br>70% by weight |

(Example 9)

| Ingredients | Amounts |
|---|---|
| N-[3-(Hexadecanamido)propyl]-N,N-dimethylammonioacetate | 3 g |
| Dipotassium glycyrrhizinate | 2 g |
| Polyvinylpyrrolidone | 3 g |
| Carboxyvinyl polymer | 0.5 g |
| Parabens | 0.15 g |
| Purified water | Total 100 g |

N-[3-(Hexadecanamido)propyl]-N,N-dimethylammonioacetate, dipotassium glycyrrhizinate and parabens were dissolved in purified water, and then polyvinylpyrrolidone and carboxyvinyl polymer were added to the solution for dissolution to give a gel.

(Example 10)

| Ingredients | Amounts |
|---|---|
| N-[3-(Cocoalkanamido)propyl]-N,N-dimethylammonioacetate | 2 g |
| Purified water | Total 100 g |

A lotion was prepared in a similar manner to that of Example 1.

(Experiment) Test of the Hair Generation Promoting Effect

Groups each of 5 male C3H mice, 7 weeks old, were used for a test of the hair generation promoting effect of alkaneamidocarboxybetaine. Dorsal hairs of the animals were shaved by an electric clipper, and 0.2 ml of the sample was applied once a day on the shaved area for 10 days. The degree of hair generation was evaluated macroscopically by the following six grades.

0 No hair generation was observed.
1 A few vellus hairs grow.
2 A few terminal hairs grow.
3 Terminal hairs grow on 50% of the shaved area.
4 Terminal hairs grow on 75% of the shaved area.
5 Terminal hairs grow on 100% of the shaved area.

The group unapplied with the sample as control was evaluated in a similar manner. 42 days after initiation of the application of the sample, results were expressed as the mean value of the evaluation.

Table 1

| Sample | Mean value of evaluation |
|---|---|
| Lotion of Example 5 | 1.4 |
| Lotion of Example 10 | 2.8 |
| Ethanol | 0 |
| Purified water | 0 |
| (No application) | 0 |

Industrial Utilization

According to the present invention, the compounds of Formula (I) or salts thereof, when topically applied to mammals, are enable to remarkably promote hair generation with extremely low skin irritation.

**Claims**

1. A method for promoting hair generation of a mammal comprising topically applying an effective amount of an alkanamidocarboxybetaine represented by the formula:

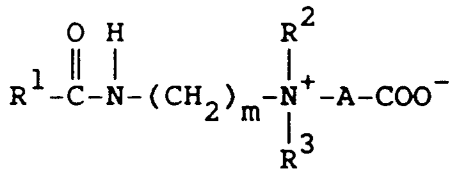

(wherein $R^1$ is an alkyl group having 5 to 25 carbon atoms, $R^2$ and $R^3$ are each an alkyl group having 1 to 4 carbon atoms, A is an alkylene group having 1 to 3 carbon atoms, or an alkylene group having 1 to 3 carbon atoms substituted by a methyl group or a hydroxyl group, and m is an integer of 1 to 3) or a salt thereof.

2. A method of Claim 1 wherein the alkanamidocarboxybetaine is N-[3-(docosanamido)propyl]-N,N-dimethylammonioacetate or N-[3-(cocoalkanamido)propyl]-N,N-dimethylammonioacetate.

3. A hair generation promoting agent comprising as an effective ingredient an alkanamidocarboxybetaine represented by the formula:

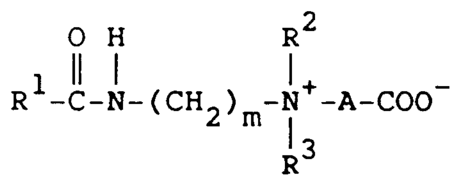

(wherein $R^1$ is an alkyl group having 5 to 25 carbon atoms, $R^2$ and $R^3$ are each an alkyl group having 1 to 4 carbon atoms, A is an alkylene group having 1 to 3 carbon atoms, or an alkylene group having 1 to 3 carbon atoms substituted by a methyl group or a hydroxyl group, and m is an integer of 1 to 3) or a salt thereof.

4. A hair generation promoting agent of Claim 3 wherein the alkanamidocarboxybetaine is N-[3-(docosanamido)propyl]-N,N-dimethylammonioacetate or N-[3-(cocoalkanamido)propyl]-N,N-dimethylammonioacetate.

5. A use of an alkanamidocarboxybetaine of represented by the formula:

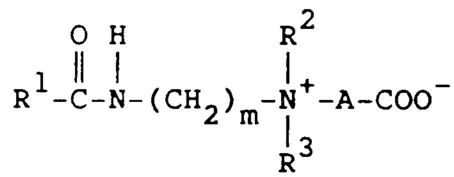

(wherein $R^1$ is an alkyl group having 5 to 25 carbon atoms, $R^2$ and $R^3$ are each an alkyl group having 1 to 4 carbon atoms, A is an alkylene group having 1 to 3 carbon atoms, or an alkylene group having 1 to 3 carbon atoms substituted by a methyl group or a hydroxyl group, and m is an integer of 1 to 3) or a salt thereof for the manufacture of a hair generation promoting agent.

**6.** A use of Claim 4 wherein the alkanamidocarboxybetaine is N-[3-(docosanamido)propyl]-N,N-dimethylammonioacetate or [3-(cocoalkanamido)propyl]-N,N-dimethylammonioacetate.

# INTERNATIONAL SEARCH REPORT

International Application No PCT/JP91/00159

**I. CLASSIFICATION OF SUBJECT MATTER** (if several classification symbols apply, indicate all) [6]

According to International Patent Classification (IPC) or to both National Classification and IPC

Int. $Cl^5$ A61K7/06

**II. FIELDS SEARCHED**

Minimum Documentation Searched [7]

| Classification System | Classification Symbols |
|---|---|
| IPC | A61K7/06 |

Documentation Searched other than Minimum Documentation to the Extent that such Documents are Included in the Fields Searched [8]

| | |
|---|---|
| Jitsuyo Shinan Koho | 1926 - 1990 |
| Kokai Jitsuyo Shinan Koho | 1971 - 1990 |

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 1-275516 (Shiseido Co., Ltd.), November 6, 1989 (06. 11. 89), (Family: none) | 1-6 |
| A | JP, A, 1-242516 (Hall Z. Raderman), September 27, 1989 (27. 09. 89), (Family: none) | 1-6 |
| A | JP, A, 63-297316 (Yutta My), December 5, 1988 (05. 12. 88) & DE, A, 3603601 & EP, A, 289639 & NO, A, 8801607 & DK, A, 8802510 & FI, A, 8801723 & AU, A, 8815805 & PT, A, 87437 & DD, A, 273379 & HU, A, T51128 | 1-6 |
| A | JP, A, 58-124712 (L'oreal), July 25, 1983 (25. 07. 83), & BE, A, 895600 & DE, A, 3301121 & GB, A, 2113245 & FR, A, 2519863 & CA, A, 1186235 & CH, A, 657523 & IT, B, 1160106 | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 10, 1991 (10. 04. 91) | April 22, 1991 (22. 04. 91) |
| **International Searching Authority** | **Signature of Authorized Officer** |
| Japanese Patent Office | |